# EUROPEAN PATENT APPLICATION

(11) **EP 4 725 509 A1**
(43) Date of publication of application: **15.04.2026**
(21) Application number: 24819349.2
(22) Date of filing: 05.06.2024
(51) Int. Cl.: A61L 2/18, A61L 9/01, A61L 9/14, A61L 101/36

(54) **DECONTAMINATION DEVICE FOR INSIDE OF ASEPTIC SEALED SPACE AND DECONTAMINATION METHOD USING SAID DEVICE**

(30) Priority: 06.06.2023 JP 2023093556
(71) Applicant: SD Biosystem Co., Ltd., Machida-shi, Tokyo 194-0013 (JP)
(72) Inventor: SAKAKI Akio, Machida-shi, Tokyo 194-0013 (JP)
(74) Representative: Cabinet Beau de Loménie
(86) International application number: PCT/JP2024/020481
(87) International publication number: WO 2024/253121

(57) **Abstract**

[Problem] To provide a highly safe device and method for decontaminating the inside of a sealed space such as in an isolator that cause less post-decontamination damage to cells and have no possibility of adsorption or re-release.

[Solution] A sealed space 12 in an isolator B and a decontamination device A are connected by a first duct 7 and a second duct 8. The decontamination device A comprises a dry fog generator 1, a temperature and humidity sensor 2, a gas collector 3, and a main control and monitoring device 4. The dry fog generator 1 generates dry fog having an average particle diameter of less than or equal to 10 µm using a decontamination agent consisting of a peracetic acid liquid mixture, and sprays the dry fog in the first duct 7 so that the concentration of the dry fog is less than or equal to 2% to fill the inside of the sealed space 12 with the dry fog. Operation of the dry fog generator 1 is stopped and operation of the gas collector 3 is started when a measured humidity from the temperature and humidity sensor 2 reaches or exceeds set humidity. The operation of the gas collector 3 is stopped and the operation of the dry fog generator 1 is started when the measured humidity drops below the set humidity.

## Description

### ( Technical Field )

The present invention relates to a decontamination device that enables decontamination of the inside of an aseptic sealed space, such as in an isolator, using a small amount of peracetic acid decontamination agent, and a decontamination method using said decontamination device.

### ( Background Art)

Mainly in manufacturing environments for sterile pharmaceutical products and products for regenerative medicine, etc., it is strictly required to ensure product quality and safety, and prevention of microbial contamination of products is an important requirement. Isolators are designed so that their insides are completely isolated from the outside and a sterile environment can be maintained. Furthermore, the isolators are widely used not only in the pharmaceutical and regenerative medicine fields but also in the biotechnology fields, including pathogenic microorganism research and food development fields, from viewpoints of ensuring data reliability and worker safety.

The isolators have a function of creating a sterility-assured environment through having a decontamination function and an aeration function for discharging decontamination agents for regular sterilization operations.

Currently, in decontamination methods for the isolators, vaporized hydrogen peroxide (VHP) is used the most because of its effectiveness, stability, and convenience. Patent Literatures 1 and 2 below are examples of using said hydrogen peroxide as the decontamination agent for a sealed space such as in an isolator.

Patent Document 1 provides an efficient and effective decontamination method by which effective sterilization can be performed in a relatively short time and a relatively limited amount of hydrogen peroxide gas is used, and discloses the decontamination method and a decontamination system using the hydrogen peroxide gas in which the generated hydrogen peroxide gas is supplied into a decontamination objective area and a temperature of said decontamination objective area is adjusted to a temperature at which the hydrogen peroxide gas is condensed.

Patent Document 2 discloses a hydrogen peroxide gas decontamination method and device capable of easily decontaminating a decontamination objective space in a small-volume housing such as a safety cabinet or an isolator, by hydrogen peroxide gas, in which the hydrogen peroxide gas decontamination method uses the hydrogen peroxide gas decontamination device including a container into which a hydrogen peroxide solution is put; a heater for heating the hydrogen peroxide solution in said container by heating the container; a temperature controller which heat-controls the hydrogen peroxide solution to between 70 and 90°C by controlling the heater; a timer connected to the temperature controller; and a neutralizing device connected to the timer and having a function of neutralizing and decomposing the hydrogen peroxide gas in the decontamination objective space, in which the hydrogen peroxide solution is heated to between 70 and 90°C by the heater, concentration of the hydrogen peroxide gas in the decontamination objective space is set to between 200 and 400ppm, and when a decontamination duration using the hydrogen peroxide gas set by the timer elapses, the heating of the hydrogen peroxide gas by the heater is stopped, the neutralizing device is automatically operated, and the hydrogen peroxide gas is decomposed to the concentration of 1 ppm or less.

### (Related Art Document)

### (Patent Document)

[Patent Document 1] JP2012-147856A
[Patent Document 2] Japanese Patent No. 6649417

### (Summary of Invention)

### (Problem to be solved by the Invention)

However, it is known that hydrogen peroxide, widely used in Patent Document 1 and 2, etc., adsorbs onto metals, resins, celluloses, glass fibers (a main material of HEPA filters is the glass fiber), etc., and is re-released. Even after aeration of a decontamination agent, a slight amount of hydrogen peroxide is re-released from the inner surface of an isolator and HEPA filter. In particular, the solubility of hydrogen peroxide in liquid is high. Therefore, in manufacturing pharmaceutical products or products for regenerative medicine, etc. using liquid formulations or cells, hydrogen peroxide dissolved in a medium damages cells. For this reason, post-decontamination management such as aeration time is important.

On the other hand, peracetic acid decontamination agents are sold in the form of a mixture containing water, acetic acid, hydrogen peroxide, and peracetic acid. This is because peracetic acid, which is a substance that cannot exist in an aqueous solution alone as it decomposes immediately, can exist in a mixture containing water, acetic acid, and hydrogen peroxide by repeating synthesis and decomposition to maintain an equilibrium state. The concentration of the hydrogen peroxide contained in this mixture is very low compared to the concentration used as a decontamination agent (mostly from the 30 to 60% aqueous solution), and ranges from about 0.8 to 10.0%, so the risk of re-release is extremely low.

In addition, in decontamination using peracetic acid, in a case of "wet corrosion" in a wet state, metals such as iron, copper and brass, which are prone to corrosion, are corroded in a short time. On the other hand, in a case of "dry corrosion" in a dry state, corrosion is so slow that there is practically no effect. Therefore, in order to prevent metal corrosion caused by condensation, it is important to establish decontamination humidity and strictly control the humidity. Furthermore, since peracetic acid has high degradability, the decontamination humidity is set relatively high at between 85 and 95% RH to enable spraying as much peracetic acid decontamination agent as possible.

A peracetic acid decontamination agent is sprayed in a state of dry fog (it is called "dry fog" because it is a droplet with a central particle diameter of about 10 µm, and it bounces back and does not wet the wall even if it collides with the wall), and decomposes into safe acetic acid, oxygen and water as soon as it evaporates from the surface of the droplet and gasifies. In addition, there are no reports of adsorption onto and re-release from metals, resins, cellulose, or glass fibers, so damage to cells is minimal.

However, even with dry fog, in a supersaturated state or in a highly concentrated state of droplets, the dry fog can condense or aggregate and wet surfaces to create a "wet corrosion" state, leading to corrosion. Therefore, humidity control and concentration control are important.

Peracetic acid exerts bactericidal activity by the action of active oxygen (a type of radical) released during its decomposition, for example, by damaging cell membranes by the action of the active oxygen on SH groups and SS bonds in microbial enzymes. In addition, radicals are highly reactive and have a short lifetime, so their decontamination action does not last long. Furthermore, since humidity is maintained even after decomposition, additional spray of a decontamination agent is not possible in a sealed space where humidity is controlled. For these reasons, it is considered that decontamination of sealed spaces using peracetic acid is difficult. In fact, although peracetic acid decontamination agents are highly safe, there are no examples of their use for decontamination of isolators in the world.

Atomization of peracetic acid decontamination agents is carried out by fogging (suction type), ultrasonic, or nozzle-compressor methods. All of them have a tank for diluted decontamination agent, and since the expiry date is short when diluted with purified water, a diluted solution remaining after decontamination is dead volume (unnecessary amount). Depending on facilities, the diluted solution may be treated as medical or industrial waste. Therefore, a mechanism to reduce the dead volume as much as possible is also important. Meanwhile, undiluted peracetic acid, which has a high concentration before dilution, is designated as a deleterious substance not for medical use because it contains 6% or more hydrogen peroxide. Automation of dilution is required to prevent human error during a dilution process and ensure safety of operators.

An emergency stop mechanism is also important because, if a humidity sensor malfunctions during decontamination, precise control is no longer possible. Furthermore, when decontaminating the inside of an isolator, it is common to maintain a slight positive pressure to prevent external contaminants from entering throughout decontamination.

For decontamination of an isolator, two ducts are used to connect a decontamination device to the isolator: one for blowing out from the decontamination device into the isolator and another for returning air from the isolator to the decontamination device. Ducts with an inner diameter of approximately 10 cm are used. However, the concentration of dry fog increases in the duct, causing the particle size of mist to increase due to agglomeration, which leads to wetting. Therefore, it is necessary to suppress the concentration of the dry fog.

An object of the present invention is to solve the above-mentioned problems, and an object of the present invention is to provide a device and a method for decontaminating a sealed space such as in an isolator under a slight positive pressure while using a peracetic acid decontamination agent of high safety with less post-decontamination damage to cells and have no possibility of adsorption and re-release, with as low dead volume as possible, preventing human error and ensuring safety by automating dilution or other processes, making an emergency stop when a humidity sensor malfunctions, and suppressing the concentration of dry fog particles to 2% or less.

### (Means for Solving the problem)

The present invention is based on a decontamination device and a decontamination method using said decontamination device of Japanese Patent No. 6285060 (hereinafter simply referred to as a "peracetic acid cycle decontamination method").

The greatest feature of said peracetic acid cycle decontamination method is that by repeatedly spraying particles consisting of a low-concentration peracetic acid mixture and collecting (dehumidifying) entire gas in an area (space) to be decontaminated, radicals are continuously produced, thereby creating a state rich in radicals (referred to as radical-rich) and killing all microorganisms. Operationally, when a measured humidity in the area to be decontaminated reaches or exceeds predetermined set humidity, spraying of dry fog is stopped and operation of a gas collector is started. When the measured humidity drops to the set humidity or below, the operation of the gas collector is stopped and operation of a dry fog generator is started. This cycle is repeated to achieve decontamination.

A decontamination device of the present invention and a sealed space of an aseptic sealed space device require maintaining sealing property for decontamination under slight positive pressure and circulating decontamination mist and gas between the aseptic sealed space and the decontamination device. Therefore, the sealed space to be decontaminated and the decontamination device are connected by two ducts, thereby incorporating a highly sealed gas circulation space into the decontamination device. Furthermore, the decontamination device comprises a dry fog generator, a humidity sensor, a gas collector, and a control device.

Specifically, the invention according to claim 1 relates to a decontamination device inside an aseptic sealed space, which is connected to the aseptic sealed space of a device having the aseptic sealed space by two ducts, and which comprises a dry fog generator, a temperature and humidity sensor, a gas collector, and a main control and monitoring device, in which
the dry fog generator comprises a purified water tank and a decontamination agent tank containing a peracetic acid mixture, a first stepping motor and a second stepping motor respectively provided in output paths of the respective tanks, with the respective output paths being connected at a merging point, a filter provided in a merged path derived from said merging point, a micro nozzle provided at an end of said merged path, and a small-sized compressor for feeding compressed air into the micro nozzle,
an air blowing fan is provided to feed dry fog sprayed by said dry fog generator through one of the two ducts to the inside of the aseptic sealed space,
an air intake fan is provided to draw gas inside the aseptic sealed space into the decontamination device through the other duct, and
the gas collector collects the gas inside the decontamination device, the temperature and humidity sensor measures a temperature and humidity inside the decontamination device, and the main control and monitoring device is configured to computer-control start or stop of operation of the dry fog generator and start or stop of operation of the gas collector according to measured values of the temperature and humidity sensor.

The invention according to claim 2 relates to the decontamination device inside an aseptic sealed space according to claim 1, in which a branch path leading to a drainage tank via a connector is provided in the merged path, and a third stepping motor for guiding liquid in the output path and the merged path to the drainage tank is provided in the branch path.

The invention according to claim 3 relates to a method for decontaminating the inside of an aseptic sealed space using a decontamination device inside the aseptic sealed space, which is connected to the sealed space of a device having the aseptic sealed space by two ducts, in which the decontamination device comprises a dry fog generator, a temperature and humidity sensor, a gas collector, and a main control and monitoring device, in which
the dry fog generator comprises a purified water tank and a decontamination agent tank containing a peracetic acid mixture, a first stepping motor and a second stepping motor, in which output paths of the respective tanks are respectively connected to a merging point via the first stepping motor and the second stepping motor, a filter provided in a merged path derived from said merging point, a micro nozzle provided at an end of said merged path, and a small-sized compressor feeding compressed air into said micro nozzle,
an air blowing fan is provided to feed dry fog sprayed by the dry fog generator through one of the two ducts to the inside of the aseptic sealed space, and an air intake fan is provided to draw gas inside the aseptic sealed space into the decontamination device through another duct,
the gas collector collects the gas inside the decontamination device, the temperature and humidity sensor measures temperature and humidity inside the decontamination device, and the main control and monitoring device is configured to computer-control start or stop of operation of the dry fog generator, start or stop of operation of the gas collector, and start or stop of operation of the air blowing fan and the air intake fan according to measured values of the temperature and humidity sensor,
set humidity inside the decontamination device is predetermined, and
the dry fog generated by the dry fog generator is fed to fill the sealed space of the device having the aseptic sealed space through one of the two ducts, and then the main control and monitoring device computer-controls so as to repeat the steps of stopping the operation of the dry fog generator and starting the operation of the gas collector to collect the gas inside the aseptic sealed space through the other duct when a measured humidity from the temperature and humidity sensor reaches or exceeds the set humidity, and stopping the operation of the gas collector and starting the operation of the dry fog generator when the measured humidity from the temperature and humidity sensor drops to the set humidity level or below.

The invention according to claim 4 relates to the method for decontaminating the inside of an aseptic sealed space according to claim 3, in which concentration of the sprayed dry fog is automatically adjusted by inputting and setting a dilution rate of the decontamination agent consisting of purified water and a peracetic acid mixture and a feed amount of a diluted solution by the first stepping motor and the second stepping motor via the main control and monitoring device.

The invention according to claim 5 relates to the method for decontaminating the inside of an aseptic sealed space according to claim 3 or 4, in which a branch path leading to a drainage tank via a connector is provided in the merged path, a third stepping motor for guiding liquid in the output path and the merged path to the drainage tank is provided in the branch path, and the liquid in the output path and the merged path after the first stepping motor and the second stepping motor is sucked into the drainage tank by the third stepping motor before starting decontamination operation.

The invention according to claim 6 relates to the method for decontaminating the inside of an aseptic sealed space according to claim 3, in which two of the temperature and humidity sensors are provided, the two temperature and humidity sensors are constantly operated, and when duration during which a difference in measured humidity between the two sensors exceeds a threshold value surpasses a predetermined time, the main control and monitoring device stops the operation of all the decontamination device.

### ( Effect of Invention )

According to the respective inventions of claims 1 and 3, the sealed space of the device having the aseptic sealed space such as an isolator can be quickly and surely decontaminated by the cycle decontamination method using peracetic acid without corroding the device or the like placed inside the isolator. In addition, unlike conventional methods using hydrogen peroxide as a decontamination agent, there is no re-release of the decontamination agent from an inside surface of the sealed space of the isolator or from the inside of a HEPA filter, and damage to cells is slight.

Furthermore, conventionally, the decontamination agent consisting of the peracetic acid mixture was diluted with the purified water and stored in the tank for the decontamination agent, and the liquid in said tank was atomized. However, since an expiration date is short in such a diluted state, the diluted liquid after the expiration date became a dead volume and was disposed of. According to the invention of claim 1, such a tank is unnecessary, and wasteful use of the decontamination agent and purified water can be reduced. In addition, the amount of use and the remaining amount of the decontamination agent and the purified water can be easily controlled. Furthermore, a compressor used to atomize the decontamination agent can be downsized.

For the purpose of minimizing the dead volume, when a micro nozzle and a small-sized compressor with a maximum pressure of 0.2 MPa were used, no droplets were observed in a duct under the following specific conditions: measured at a flight distance of 30 cm using the laser diffraction particle size distribution analyzer HELOS-KR, and with a measured concentration of 2% or less determined by light shielding rate.

According to the respective inventions of claims 2 and 5, the liquid remaining in the output path and the merged path after the first stepping motor and the second stepping motor can be sucked into the drainage tank by the third stepping motor for cleaning the tubes such as the output path and the merged path prior to the start of the decontamination operation, whereby an expired decontamination agent and purified water do not remain in the paths when the dry fog generator is operated anew, and the decontamination operation can always be carried out using fresh decontamination agent and purified water.

According to the invention of claim 4, by inputting and setting the dilution ratio of the decontamination agent to be fed to the micro nozzle and the amount of the diluted solution to be fed, the concentration of the dry fog can be adjusted in the duct of a sealed system, and it is possible to prevent the dry fog from being agglomerated in the duct to increase the mist particle size and wet the inner wall of the duct.

According to the invention of claim 6, it is possible to prevent corrosion of the device placed inside the isolator even if the temperature and humidity sensor malfunctions during decontamination by stopping the operation of the dry fog generator and starting the operation of the gas collector by the main control and monitoring device when the duration during which the difference in the measured humidity between the two temperature and humidity sensors exceeds the threshold value surpasses the predetermined time.

### ( Brief Description of Drawings )

FIG.1 is a schematic configuration view of a decontamination device for an aseptic sealed space according to Embodiment 1 of the present invention.
FIG. 2 is a schematic configuration view showing a state in which the decontamination device for the aseptic sealed space and a device having the aseptic sealed space according to Embodiment 1 of the present invention are connected by ducts.
FIG. 3 is a configuration view showing a dry fog generator of the decontamination device for the aseptic sealed space according to Embodiment 1 of the present invention.
FIG. 4 is a configuration view of mounting locations of an air blowing fan of a micro nozzle and an air intake fan of a gas collection location of the dry fog generator in the decontamination device for the aseptic sealed space according to Embodiment 1 of the present invention.

### ( Mode for Carrying out the Invention )

### (Embodiment example 1)

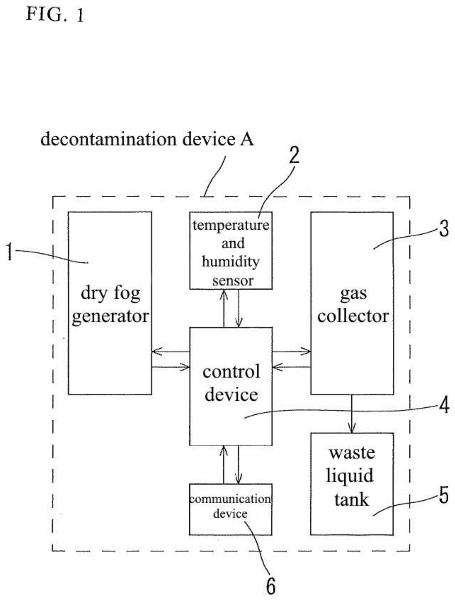

A decontamination device A for decontaminating the inside of an aseptic sealed space according to Embodiment example 1 of the present invention will be described with reference to FIGs. 1 to 4.

First, as shown in FIG. 1, the decontamination device A is configured such that a dry fog generator 1, a temperature and humidity sensor 2, and a gas collector 3 are computer-controlled by a main control and monitoring device 4. The dry fog generator 1 uses a peracetic acid mixture containing low-concentration peracetic acid, water, acetic acid, and hydrogen peroxide as a decontamination agent to generate dry fog consisting of particles with an average diameter of 10 µm. The gas collector 3 includes a dehumidifier for collecting a gas consisting of acetic acid, water and active oxygen generated by decomposition of said peracetic acid mixture of the dry fog particles in an aseptic sealed space described below, and the whole gas of the peracetic acid mixture. The gas collector 3 is provided with a waste liquid tank 5 for storing moisture of the gas collected by said gas collector 3.

The main control and monitoring device 4 performs on/off control of the dry fog generator 1 and the gas collector 3 by a computer based on temperature and humidity information measured by the temperature and humidity sensor 2. The main control and monitoring device 4 also has a monitoring function. Furthermore, said main control and monitoring device 4 also performs on/off control of an air blowing fan 34 and an air intake fan 36, described below, by the computer. Said main control and monitoring device 4 transmits information to an external control device(computer) via the communication device 6, either by wired or wireless means, and can also be controlled by said external control device.

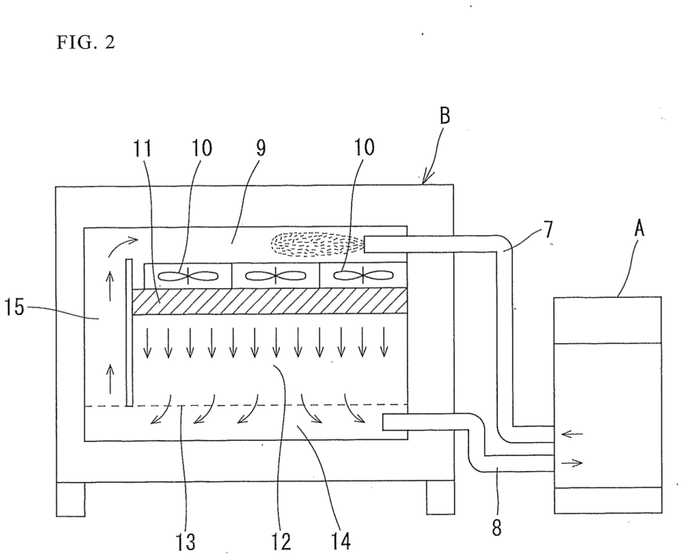

Said decontamination device A is used for decontamination of the inside of a sealed space of an isolator B as a device having an aseptic sealed space. Specifically, as shown in FIG. 2, the decontamination device A and the isolator B are connected by two ducts; a first duct 7 and a second duct 8.

The first duct 7 feeds the dry fog sprayed from the decontamination device A to a preliminary chamber 9 above the isolator B by the air blowing fan 34 described below. Then, the dry fog is fed from said preliminary chamber 9 to a sealed space 12 through a HEPA filter 11 by a fan 10 to decontaminate the sealed space 12.

In the sealed space 12, the peracetic acid in the dry fog consisting of the peracetic acid mixture is immediately decomposed. The peracetic acid kills microorganisms by, for example, the action of active oxygen (a type of radical) released during its decomposition on SH groups or SS bonds of microbial enzymes, thereby disrupting cell membranes. In addition, radicals are highly reactive and have a short lifetime, so their decontamination action does not last long. Furthermore, since humidity is maintained even after the decomposition, additional spray of the decontamination agent is not possible in the sealed space where the humidity is controlled.

Therefore, the temperature and humidity sensor 2 measures the humidity, and when the measured humidity reaches or exceeds predetermined set humidity, the main control and monitoring device 4 stops operation of the dry fog generator 1 and starts operation of the gas collector 3. The air intake fan 36 described below is also operated. This dehumidifies the inside of a sealed circulation space consisting of the sealed space 12, the first duct 7, the second duct 8, and the space inside the decontamination device A. When the measured humidity from the temperature and humidity sensor 2 drops, the operation of the gas collector 3 is stopped, and the operation of the dry fog generator 1 is restarted.

By repeating this cycle operation, radicals are continuously produced in the sealed space 12 to create a state rich in radicals (called radical-rich), whereby all microorganisms can be killed.

Then, air in the sealed space 12 of the isolator B passes through a grating 13 at the lower part and enters a lower passage 14, where part of the air enters the second duct 8 and is taken into the gas collector 3 in the decontamination device A. Another part of the air passes through the lower passage 14 and a vertical passage 15 to reach the preliminary chamber 9, and again passes through the HEPA filter 11 by the fan 10 to enter the sealed space 12. This sealed circulation space is maintained at a slight positive pressure.

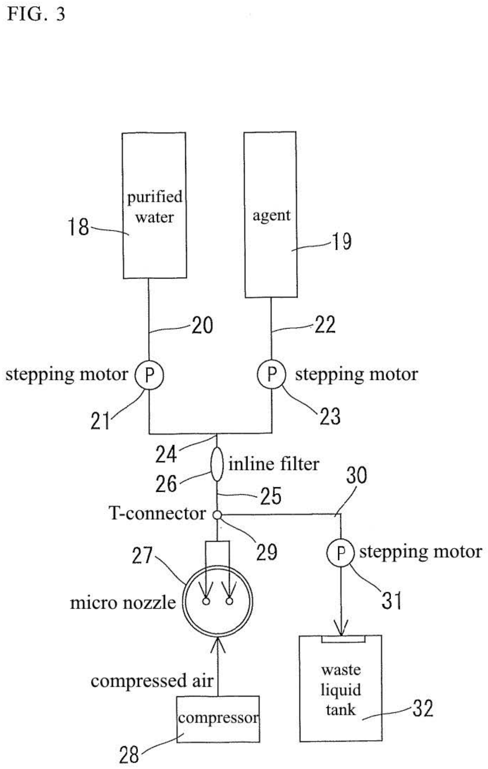

Next, a specific example of the dry fog generator 1 is shown in FIG. 3. The dry fog generator 1 comprises a purified water tank 18 and a decontamination agent tank 19 containing the peracetic acid mixture. A first stepping motor 21 is provided in an output path 20 of the purified water tank 18, and a second stepping motor 23 is provided in an output path 22 of the decontamination agent tank 19. These output paths 20 and 22 are merged at a merging point 24, and an inline filter 26 is provided in a merged path 25 derived from said merging point 24. The first stepping motor 21 and the second stepping motor 23 can be turned on and off by computer-control by the main control and monitoring device 4.

A distal end of said merged path 25 is connected to a micro nozzle 27, into which compressed air flows from a compressor 28, so that a mixture (diluted solution) of the purified water and decontamination agent can be sprayed from said micro nozzle 27 as dry fog having an average particle diameter of 10 µm.

Further, a T-connector 29 is provided in the merged path 25 to provide a branch path 30. A third stepping motor 31 provided in said branch path 30 can suck up the purified water, the decontamination agent, and the mixture thereof remaining in the output paths 20 and 22 after the first stepping motor 21 and the second stepping motor 23 and in the merged path 25, and store them in a drainage tank 32. The third stepping motor 31 can also be computer-controlled by the main control and monitoring device 4. The output paths 20 and 22, the merged path 25, and the branch path 30 are all formed of tubes or small diameter pipes.

As described above, in the method of the present invention, a small nozzle (micro nozzle 27) having no dead volume is used, and a diluted liquid tank is not provided. The decontamination agent and purified water are combined in a narrow tube immediately before use, and passed through the in-line filter 26 to filter out contaminants and mix, allowing dead volume to be reduced.

The compressor 28 is a small-sized one having no tank for compressed air. Since the effective maximum pressure of the small-sized compressor is approximately 0.2 MPa, the particle diameter of the dry fog sprayed from the micro nozzle 27 is about 11.0 ± 1.0 µm in SMD (calculated particle size based on surface area), whose measured concentration is approximately 10.9% under natural water supply. At this concentration, liquid accumulates in the duct, radicals are consumed at the liquid surface, thereby reducing the decontamination effect. To solve this problem, flow rates of the decontamination agent and the purified water are controlled by the first stepping motor 21 and the second stepping motor 23, respectively. This allows the concentration of the dry fog to be maintained at 2% or less by accurately controlling the spray volume, and enables decontamination in a dry state even with low-pressure compressed air.

Simultaneously, operation of the first and second stepping motors 21 and 23 can be controlled by inputting and setting the dilution ratio of the peracetic acid mixture with purified water and the feeding amount of said diluted solution into the computer of the main control and monitoring device 4, thereby automatically adjusting the concentration of the dry fog to be sprayed. This makes it possible to prevent human error in dilution operation and ensure safety of operators. In addition, as a parameter for decontamination of the sealed space 12, it is possible to control decontamination time after reaching the set humidity for decontamination, as well as control the amount of the purified water and the decontamination agent used taking into account the effect of temperature rise. Furthermore, it is possible to control remaining amounts of purified water and the decontamination agent, which makes it possible to prevent erroneous use of expired materials by setting expiration dates.

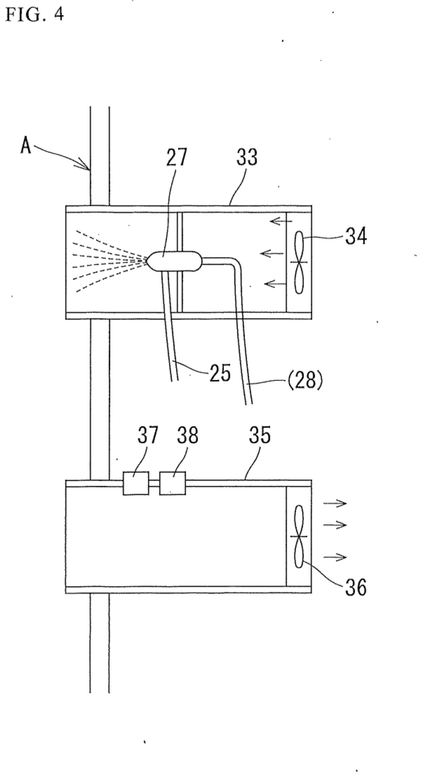

Furthermore, as shown in FIG. 4, the micro nozzle 27 of the dry fog generator 1 is supported and fixed within an air blowing port pipe 33 that penetrates the outer wall of the decontamination device A, and the air blowing fan 34 is provided in the air blowing port pipe 33 behind said micro nozzle 27. The first duct 7 is connected at the front end of said air blowing port pipe 33.

In addition, an air intake port pipe 35 is provided alongside the air blowing port pipe 33, penetrating the outer wall of the decontamination device A. The second duct 8 is connected to a front end of said air intake port pipe 35, and an air intake fan 36 is provided inside a rear end thereof. The air blowing fan 34 and the air intake fan 36 operate constantly during the decontamination operation, and a circulation path from the decontamination device A toward the first duct 7, the isolator B, the second duct 8, and back to the decontamination device A is formed, through which dry fog, gas, and radicals circulate. And it increases the radicals overall.

Furthermore, temperature and humidity sensors 37 and 38 are provided in said air intake port pipe 35 of the decontamination device A to measure a temperature and humidity inside said air intake port pipe 35. These two temperature and humidity sensors 37 and 38 are calibrated, and thus the measured values are synchronized with each other with small differences. Therefore, it is configured such that a threshold value for the difference in measured humidity (RH%) between these temperature and humidity sensors and duration of deviation from the threshold value are predetermined, and when the duration of deviation from the threshold value is exceeded, an abnormality of the temperature and humidity sensors is detected, an alarm is issued, the operation of the dry fog generator is stopped, and the operation of all the decontamination device.

In the above embodiment example 1, it is described with an example where the decontamination device A decontaminates the sealed space 12 of the isolator B. However, the decontamination target of this invention is not limited to the isolator B and can be applied to other devices having an aseptic sealed space.

Furthermore, in the above embodiment example 1, the decontamination device A and the isolator B are connected by the duct, but a pipe such as a blower pipe may be used instead of the duct. The decontamination device A may be independent of the device having the aseptic sealed space to be decontaminated, or the decontamination device A may be incorporated in the device.

### ( Description of Reference Numerals and Symbols )

- A: decontamination device
- B: isolator
- 1: dry fog generator
- 2: temperature and humidity sensor
- 3: gas collector
- 4: control device
- 5: waste liquid tank
- 6: communication device
- 7: first duct
- 8: second duct
- 9: preliminary chamber
- 10: fan
- 11: HEPA filter
- 12: sealed space
- 13: grating
- 14: lower passage
- 15: vertical passage
- 18: purified water tank
- 19: decontamination agent tank
- 20: output path
- 21: first stepping motor
- 22: output path
- 23: second stepping motor
- 24: merging point
- 25: merged path
- 26: inline filter
- 27: micro nozzle
- 28: compressor
- 29: T-connector
- 30: branch path
- 31: third stepping motor
- 32: drainage tank
- 33: air blowing port pipe
- 34: air blowing fan
- 35: air intake port pipe
- 36: air intake fan
- 37: temperature and humidity sensor
- 38: temperature and humidity sensor

## Claims

1. A decontamination device inside an aseptic sealed space, which is connected to the aseptic sealed space of a device having the aseptic sealed space by two ducts, and which comprises a dry fog generator, a temperature and humidity sensor, a gas collector, and a main control and monitoring device, wherein
the dry fog generator comprises a purified water tank and a decontamination agent tank containing a peracetic acid mixture, a first stepping motor and a second stepping motor respectively provided in output paths of the respective tanks, with the respective output paths being connected at a merging point, a filter provided in a merged path derived from said merging point, a micro nozzle provided at an end of said merged path, and a small-sized compressor for feeding compressed air into said micro nozzle,
an air blowing fan is provided to feed dry fog sprayed by said dry fog generator through one of the two ducts to the inside of the aseptic sealed space,
an air intake fan is provided to draw gas inside the aseptic sealed space into the decontamination device through the other duct, and
the gas collector collects the gas inside the decontamination device, the temperature and humidity sensor measures a temperature and humidity inside the decontamination device, and the main control and monitoring device is configured to computer-control start or stop of operation of the dry fog generator and start or stop of operation of the gas collector according to measured values of the temperature and humidity sensor.

2. The decontamination device inside an aseptic sealed space according to claim 1, wherein a branch path leading to a drainage tank via a connector is provided in the merged path, and a third stepping motor for guiding liquid in the output path and the merged path to the drainage tank is provided in the branch path.

3. A method for decontaminating the inside of an aseptic sealed space using a decontamination device inside the aseptic sealed space, which is connected to the sealed space of a device having the aseptic sealed space by two ducts, wherein the decontamination device comprises a dry fog generator, a temperature and humidity sensor, a gas collector, and a main control and monitoring device, wherein
the dry fog generator comprises a purified water tank and a decontamination agent tank containing a peracetic acid mixture, a first stepping motor and a second stepping motor, in which output paths of the respective tanks are respectively connected to a merging point via the first stepping motor and the second stepping motor, a filter provided in a merged path derived from said merging point, a micro nozzle provided at an end of said merged path, and a small-sized compressor for feeding compressed air into said micro nozzle,
an air blowing fan is provided to feed dry fog sprayed by said dry fog generator through one of the two ducts to the inside of the aseptic sealed space, and an air intake fan is provided to draw gas inside the aseptic sealed space into the decontamination device through another duct,
the gas collector collects the gas inside the decontamination device, the temperature and humidity sensor measures temperature and humidity inside the decontamination device, and the main control and monitoring device is configured to computer-control start or stop of operation of the dry fog generator and start or stop of operation of the gas collector according to measured values of the temperature and humidity sensor,
set humidity inside the decontamination device is predetermined, and
the dry fog generated by the dry fog generator is fed to fill the sealed space of the device having the aseptic sealed space through one of the two ducts, and then the main control and monitoring device computer-controls so as to repeat the steps of stopping the operation of the dry fog generator and starting the operation of the gas collector to collect the gas inside the aseptic sealed space through the other duct when a measured humidity from the temperature and humidity sensor reaches or exceeds the set humidity, and stopping the operation of the gas collector and starting the operation of the dry fog generator when the measured humidity from the temperature and humidity sensor drops to the set humidity level or below.

4. The method for decontaminating the inside of an aseptic sealed space according to claim 3, wherein concentration of the sprayed dry fog is automatically adjusted by inputting and setting a dilution rate of the decontamination agent consisting of purified water and a peracetic acid mixture and a feed amount of a diluted solution by the first stepping motor and the second stepping motor via the main control and monitoring device.

5. The method for decontaminating the inside of an aseptic sealed space according to claim 3 or 4, wherein a branch path leading to a drainage tank via a connector is provided in the merged path, a third stepping motor for guiding liquid in the output path and the merged path to the drainage tank is provided in the branch path, and the liquid in the output path and the merged path after the first stepping motor and the second stepping motor is sucked into the drainage tank by the third stepping motor before starting decontamination operation.

6. The method for decontaminating the inside of an aseptic sealed space according to claim 3, wherein two of the temperature and humidity sensors are provided, the two temperature and humidity sensors are constantly operated, and when duration during which a difference in measured humidity between the two sensors exceeds a threshold value surpasses a predetermined time, the main control and monitoring device stops the operation of all the decontamination device.
